# EUROPÄISCHE PATENTANMELDUNG

(11) **EP 2 198 796 A1**
(43) Veröffentlichungstag der Anmeldung: **23.06.2010**
(21) Anmeldenummer: 08405311.5
(22) Anmeldetag: 19.12.2008
(51) Int. Cl.: A61B 17/86, A61B 19/00

(54) **Knochenschraube**

(71) Anmelder: Sepitec Foundation, 9490 Vaduz (LI)
(72) Erfinder: Fiechter, Meinrad, 3110 Münsingen (CH); Magerl, Friedrich, 9011 St. Gallen (CH); Wieling, Ronald, 9453 Eichberg (CH)
(74) Vertreter: Groner, Manfred

(57) **Zusammenfassung**

Die Knochenschraube besitzt einen Schraubenschaft (15), der eine Spitze (13) aufweist und der konzentrisch zu einer Schraubenlängsachse (27) und der in einen Knochen- oder Knochenteil zu verankern ist. Am Schraubenschaft (15) ist ein Schraubenkopf (14) befestigt. Der Schraubenschaft (15) ist im Wesentlichen aus einem röntgentransparenten Kunststoff hergestellt. Ein Teil (5, 7, 9, 24) des Schraubenschaftes (15) ist aus einem anderen Werkstoff hergestellt als derjenige eines Kerns (3, 30), welcher im Wesentlichen den Schraubenschaft (15) bildet. Vorzugsweise ist der wenigstens eine lasttragende Bereich (5, 7, 9, 24) aus Metall oder Kunststoff, beispielsweise PEEK hergestellt.

## Beschreibung

Die vorliegende Erfindung betrifft eine Knochenschraube mit einem Schraubenschaft, der eine Spitze aufweist und konzentrisch zu einer Schraubenlängsachse ist und der in einen Knochen oder Knochenteil zu verankern ist und mit einem am Schraubenschaft befestigten und Angriffsmittel aufweisenden Schraubenkopf.

Knochenschrauben dieser Art sind seit langem bekannt. Sie dienen beispielsweise zur osteosynthetischen Knochenfixierung. Solche Schrauben dienen hierbei insbesondere zum Verankern einer Platte oder eines Verbindungsstabes an einer Wirbelsäule. Die Schrauben werden hierbei wesentlich beansprucht, beispielsweise durch eine Klemmverbindung zwischen der Schraube und einem Verbindungsstab oder zu einer Platte. Eine Knochenschraube dieser Art ist beispielsweise aus der US 5, 466,237 bekannt geworden. Diese dient zum Befestigen eines Verbindungsstabes an einer Wirbelsäule. Der Verbindungsstab wird hierbei zwischen dem Schraubenkopf und einer Mutter festgeklemmt.

Die EP 0 507 162 A offenbart eine Knochenschraube, die zum Befestigen einer Knochenplatte an einer Wirbelsäule dient. Mittels einer Hülse wird die Knochenschraube in der Knochenplatte gehalten.

Die EP 1 191 891 A offenbart eine Knochenschraube, die einen axial zweistückigen Schraubenkopf besitzt. Dadurch soll das Einführen eines Schrauendrehers in den Schraubenkopf vereinfacht werden.

Die genannten Knochenschrauben sind aus Metall, insbesondere Titan hergestellt. Diese besitzen eine hohe Festigkeit, sodass sie beim Einschrauben und beim Verbinden mit einem Implantat hochbelastbar sind. Nachteilig ist hier jedoch die mangelnde Röntgentransparenz.

Der Erfindung liegt die Aufgabe zugrunde, eine Knochenschraube der genannten Art zu schaffen, die weitergehend oder zumindest bereichsweise röntgentransparent ist, die aber dennoch funktionssicher ist.

Die Aufgabe ist bei einer gattungsgemässen Knochenschraube dadurch gelöst, dass der Schraubenschaft im Wesentlichen aus einem röntgentransparenten Kunststoff hergestellt ist und dass wenigstens ein lastentragender Bereich einen Teil aufweist, wobei dieser Teil aus einem anderen Werkstoff hergestellt ist als diejenige eines Kerns, welcher im Wesentlichen den Schraubenschaft bildet. Die Röntgentransparenz der erfindungsgemässen Knochenschraube ergibt sich durch die Herstellung des Schraubenschaftes aus einem röntgentransparenten Kunststoff, beispielsweise PEEK oder faserverstärktem PEEK. Faserverstärktes PEEK und PEEK sind röntgentransparent und besitzt eine vergleichsweise hohe Festigkeit. Faserverstärktes PEEK ist jedoch zugleich vergleichsweise spröd. Ein Schraubenschaft aus faserverstärktem PEEK ist trotz dieser Sprödigkeit jedoch möglich, da wenigstens ein lasttragender Bereich ein Teil aus einem anderen Werkstoff aufweist. Dieser Werkstoff ist Metall oder PEEK. Dieser Teil ist vorzugsweise am Schraubenkopf angeordnet und weist die genannten Angriffsmittel auf. Ist der genannte Teil aus Metall, so ist die Knochenschraube lediglich im Bereich des Schraubenkopfes nicht röntgentransparent. Eine solche Schraube kann wie bei einer üblichen Schraube aus Titan am Schraubenkopf beispielsweise mittels eines Schraubendrehers eingedreht und auch geklemmt werden. Ein lasttragender Bereich ist ein Bereich der besonders beansprucht ist wie insbesondere der Schraubenkopf und/oder die Schraubenspitze.

Nach einer Weiterbildung der Erfindung ist vorgesehen, dass der genannte Teil an der Spitze des Schraubenschaftes angeordnet ist. Dieser Teil ist insbesondere mit einem Gewinde versehen und ermöglicht hohe Bohr- oder Gewindeschneideeigenschaften. Die Bohrspitze kann zudem selbstschneidend ausgebildet werden. Trotzdem ist die erfindungsgemässe Knochenschraube im Bereich des Schraubenschaftes weitgehend röntgentransparent.

Der genannte Teil ist gemäss einer Weiterbildung der Erfindung hülsenförmig ausgebildet. Ein solcher hülsenförmiger Teil kann beispielsweise durch Presssitz oder durch Verkleben mit dem Schraubenschaft verbunden werden. Die Hülse ist gemäss einer Weiterbildung der Erfindung zur Verbesserung der Abscherfestigkeit vorgesehen. Diese Hülse vorzugsweise in einem mittleren Bereich des Schraubenschaftes zwischen dem Schraubenkopf und der Spitze angeordnet.

Vorzugsweise ist der Teil am Schraubenkopf angeordnet. Denkbar ist aber auch eine Ausführung, bei welcher ein erster Teil am Schraubenkopf und ein zweiter Teil an der Spitze des Schraubenschaftes angeordnet sind. Einerseits ergeben sich dadurch sehr gute Bohr- und Gewindeschneideeigenschaften und andererseits eine hohe Belastbarkeit der Knochenschraube am Schraubenkopf. Auch in diesem Fall ist der Schraubenschaft weitgehend röntgentransparent.

Ausführungsbeispiele der Erfindung werden nachfolgend anhand der Zeichnung näher erläutert:

Es zeigen:

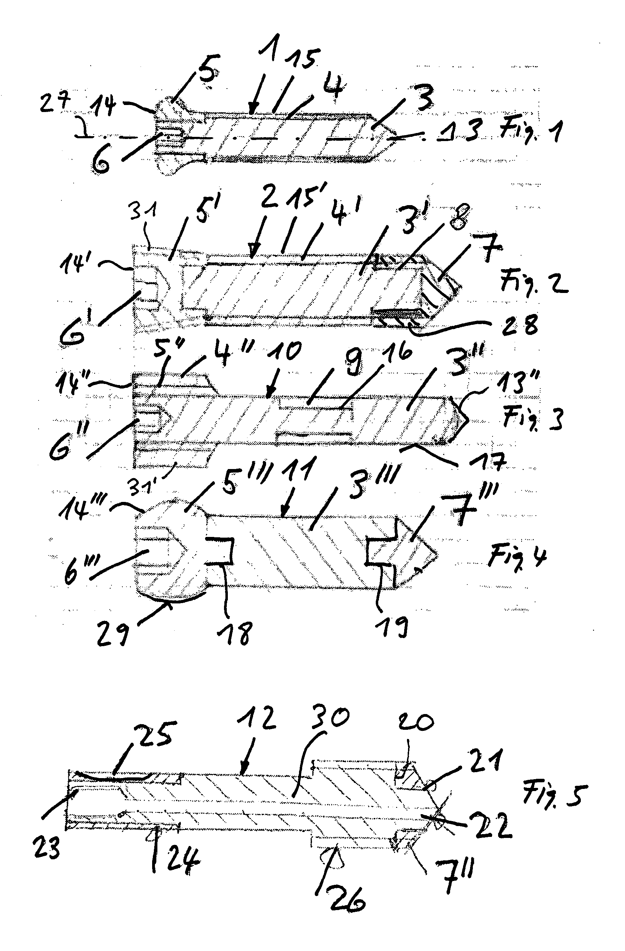

- Figuren 1 - 5: Jeweils schematisch Längsschnitte durch erfindungsgemässe Schrauben.

Die Figur 1 zeigt eine Schraube 1, die einen Kern 3 aufweist, welcher den Schraubenschaft bildet. Dieser besitzt im Wesentlichen auf seiner gesamten Länge ein Gewinde 4 als Aussengewinde. Der Schaft 15 ist koaxial zu einer Schraubenlängsachse 27. Der Kern 3 besteht aus einem röntgentransparenten Kunststoff, beispielsweise faserverstärktem PEEK und insbesondere kohlenfaserverstärktem PEEK oder PEEK. Die Fasern können lange ausgerichtete Fasern oder auch kurze Fasern sein. Der Schaft 15 ist wie ersichtlich konzentrisch zu einer Schraubenlängsachse 27. Es sind aber auch andere röntgentransparente und biokompatible Kunststoffe wie Polycarbonat, Ployaethylen, PEK oder PEEKEK denkbar.

Gegenüber der Spitze 13 ist am Schaft 15 ein Kopf 14 befestigt, der einen hülsenförmigen Teil 5 besitzt. Dieser Teil 5 ist fest mit dem Kern 3 verbunden, beispielsweise mittels eines Presssitzes oder durch Verkleben. Dieser Teil 5 besteht aus einem Metall, beispielsweise aus Titan oder Stahl. Besteht der Kern 3 aus kohlenfaserverstärktem PEEK, so kann der Teil 5 aus PEEK bestehen. Verstärkende Fasern sind in diesem Fall lediglich im Kern 3 vorgesehen. Der Teil 5 ist beispielsweise im Fall einer Klemmung an einer Platte oder einem Verbindungsstab der lasttragende Bereich. Zum Einschrauben der Schraube 1 in einen Knochen oder einen Knochenteil besitzt der Kopf 14 einen Innenangriff 6, beispielsweise in der Form einer mehrkantigen Vertiefung. Grundsätzlich kann der Angriff jedoch auch aussen am Teil 5 erfolgen.

Die Figur 2 zeigt eine Schraube 2, die als selbstschneidende Schraube ausgebildet ist. Sie besitzt ebenfalls einen Kern 3', der aus faserverstärktem PEEK oder PEEK hergestellt ist. Der Kern 3' besitzt ein Gewinde 4', das ebenfalls ein Aussengewinde ist und das dazu dient, die Schraube 2 in einem Knochen oder einem Knochenteil zu verankern. Die Spitze des Schaftes 15' wird hier durch eine Spitze 7 gebildet, die aus einem anderen Material hergestellt ist als der Kern 3'. Die Spitze 7 kann ein Gewinde 28 aufweisen, das ein Aussengewinde und selbstschneidend sein kann. Das Gewinde 28 ergänzt das Gewinde 4' im Bereich der Spitze. Die Spitze 7 ist vorzugsweise aus einem vergleichsweise harten Material, beispielsweise Titan oder Stahl hergestellt. Die Befestigung der Spitze 7 mit dem Kern 4' erfolgt beispielsweise ebenfalls durch Presssitz, Kleben oder beispielsweise Aufschrauben. Es sind aber auch andere Verbindungsarten möglich.

Der Schraubenkopf 14' wird hier durch eine Hülse 5' gebildet, die ebenfalls aus einem anderen Material hergestellt ist als der Kern 3'. Die Hülse 15' ist fest mit dem Kern 3' verbunden. Sie besteht beispielsweise aus Metall, insbesondere Titan oder im Fall eines Kernes 3' aus faserverstärktem PEEK aus PEEK. Die Hülse 5' ist mit einem Innenangriff 6' versehen. Der Schraubenkopf 14' weist ein konisches Gewinde 31 auf, das zur Befestigung in einer korrespondierenden Öffnung einen hier nicht gezeigten Platte geeignet ist.

Die Figur 3 zeigt eine Schraube 10, die als translaminarer Pin geeignet ist. Sie besitzt einen Kern 3" aus faserverstärktem PEEK oder PEEK. Etwa mittig zwischen einer Spitze 13" und einem Kopf 14" ist in den Kern 3" eine umlaufende Ausnehmung 16 oder Nut eingearbeitet, in welche eine Hülse 9 eingesetzt wird. Diese ist zumindest axial fixiert und besteht aus einem anderen Werkstoff als der Kern 3". Die Hülse 9 dient zur Erhöhung der Abscherfestigkeit im mittleren Bereich den Kerns 3". Diese Hülse 9 kann aber auch an einer anderen Stelle des Kerns 3" angeordnet sein, beispielsweise näher bei der Spitze 3" oder näher beim Kopf 14". Die Hülse 9 besteht vorzugsweise aus einem geeigneten Metall, beispielsweise aus Stahl oder Titan. Bezüglich einer Umfangsfläche 17 ist diese Hülse 9 bündig. Der Kopf 14" besitzt eine Hülse 5", die ein Aussengewinde 14" besitzt. Diese Hülse 5" ist ersichtlich vorzugsweise zylindrisch ausgebildet und mit dem Kern 3" fest verbunden, beispielsweise mit diesem verklebt. Der Kopf 14" besitzt ebenfalls einen Innenangriff 6".

Die Figur 4 zeigt eine Schraube 11, die einen Kern 3"' aufweist, der ebenfalls aus PEEK oder faserverstärktem PEEK hergestellt ist. An einem vorderen Ende besitzt der Kern 3"' eine Ausnehmung 19, in die eine Spitze 7"' eingesetzt ist, die aus einem wesentlich härteren Werkstoff hergestellt ist, dass derjenige des Kerns 3"'.

Der Kern 3"' besitzt eine weitere Ausnehmung 18, in die eine Hülse 5"' eingesetzt und fest mit dem Kern 3"' verbunden ist. Die Hülse 5"' bildet einen Kopf 14"', der eine kugelförmige Aussenseite 29 und einen Innenangriff 6"' aufweist. Diese Schraube 11 ist vorzugsweise als selbstschneidende Schraube ausgebildet. Zwischen der Spitze 7"' und dem Kopf 14"' ist diese röntgentransparent. Der Kopf 14"' eignet sich beispielsweise für eine polyaxiale Lagerung in einem Petikularsystem. Beispielsweise kann der Kopf 14"' in einer entsprechenden Ausnehmung in einer Platte gelagert und festklemmbar sein. Die Hülse 5"' besteht beispielsweise aus Titan oder einem geeigneten Stahl.

Die Figur 5 offenbart eine Schenkelhalsschraube 12, die wie ersichtlich in Längsrichtung gesehen abgestuft ist und an einem vorderen Ende einen grösseren Aussendurchmesser mit einem zylindrischen Gewinde 26 aufweist. Sie besitzt einen Kern 30, der eine sich in Achsialrichtung erstreckende Kannulierung 22 besitzt. Diese Kannulierung 22 ist an beiden Enden des Kerns 30 offen. Am vorderen zugespitzten Ende besitzt der Kern 30 eine umlaufende Ausnehmung 20, in die eine hülsenförmige Spitze 7" eingesetzt ist. Diese Spitze 7" ist fest mit dem Kern 30 verbunden, beispielsweise verklebt. Denkbar ist aber auch beispielsweise eine Verbindung 21, die eine Pressverbindung oder Schraubenverbindung oder dergleichen ist. Gegenüber der Spitze 7" ist eine Hülse 24 angeordnet, welche ebenfalls fest mit dem Kern 30 verbunden ist. Diese Hülse 24 besitzt eine Vertiefung 25, die beispielsweise als Nut ausgebildet ist, welche sich in Axialrichtung erstreckt und an welcher die Schraube 12 gegen eine Rotation gesichert werden kann. Die Spitze 7" und die Hülse 24 sind ebenfalls aus einem anderen Material hergestellt als der Kern 30. Es sind die Materialien bzw. Werkstoffe vorgesehen, die bereits oben erwähnt sind. Insbesondere bestehen die Spitze 7" und die Hülse 24 aus einem Werkstoff, der wesentlich härter ist als der Werkstoff des Kerns 30. Die in den Figuren 1 bis 5 gezeigten Köpfe 14 bis 14"' können jeweils grundsätzlich mit jedem der gezeigten Schäfte und Spitzen verbunden sein. Die in Figur 5 gezeigte Kannulierung 22 ist zudem auch bei den in den Figuren 1 bis 4 gezeigten Schrauben denkbar. Selbstverständlich können die Schrauben auch an sich geeignete Marker, beispielsweise aus Tantalfasern, Bariumsulfat aufweisen. Zudem sind Beschichtungen denkbar, beispielsweise aus Titan oder Hydroxioapatit.

### BEZUGSZEICHENLISTE

- 1: Schraube
- 2: Schraube
- 3: Kern
- 4: Gewinde
- 5: Hülse
- 6: Innenangriff
- 7: Spitze
- 8: Verbindung (Gewinde)
- 9: Hülse
- 10: Schraube
- 11: Schraube
- 12: Schraube
- 13: Spitze
- 14: Kopf
- 15: Schaft
- 16: Ausnehmung
- 17: Umfang
- 18: Ausnehmung
- 19: Ausnehmung
- 20: Ausnehmung
- 21: Verbindung
- 22: Kannulierung
- 23: Innenangriff (Gewinde)
- 24: Hülse
- 25: Vertiefung
- 26: Gewinde
- 27: Schraubenlängsachse
- 28: Gewinde
- 29: Aussenseite
- 30: Kern
- 31: Gewinde

## Patentansprüche

1. Knochenschraube mit einem Schraubenschaft (15), der eine Spitze (13) aufweist und konzentrisch zu einer Schraubenlängsachse (27) ist und der in einen Knochen- oder Knochenteil zu verankern ist und mit einem am Schraubenschaft (15) befestigten und Angriffsmittel (6) aufweisenden Schraubenkopf (14), **dadurch gekennzeichnet, dass** der Schraubenschaft (15) im Wesentlichen aus einem röntgentransparenten Kunststoff hergestellt ist und dass wenigstens ein lasttragender Bereich einen Teil (5, 7, 9, 24) aufweist, wobei dieser Teil (5, 7, 9, 24) aus einem anderen Werkstoff hergestellt ist als derjenige eines Kerns (3, 30), welcher im Wesentlichen den Schraubenschaft (15) bildet.

2. Knochenschraube nach Anspruch 1, **dadurch gekennzeichnet, dass** der wenigstens eine lasttragende Bereich (5, 7, 9, 24) aus Metall oder Kunststoff, beispielsweise PEEK hergestellt ist.

3. Knochenschraube nach Anspruch 1 oder 2, **dadurch gekennzeichnet, dass** der genannte Teil am Schraubenkopf (14) angeordnet ist.

4. Knochenschraube nach einem der Ansprüche 1 bis 3, **dadurch gekennzeichnet, dass** der genannte Teil (7) wenigstens bereichsweise eine Spitze bildet.

5. Knochenschraube nach einem der Ansprüche 1 bis 4, **dadurch gekennzeichnet, dass** der genannte Teil (5, 7, 9, 24) hülsenförmig ausgebildet ist.

6. Knochenschraube nach einem der Ansprüche 1 bis 5, **dadurch gekennzeichnet, dass** der genannte Teil aus Titan oder Stahl hergestellt ist.

7. Knochenschraube nach einem der Ansprüche 1 bis 5, **dadurch gekennzeichnet, dass** der genannte Teil (9) zwischen dem Schraubenkopf (14) und der Spitze (7) angeordnet ist und vorgesehen ist, die Abscherfestigkeit in diesem Bereich zu erhöhen.

8. Knochenschraube nach einem der Ansprüche 1 bis 6, **dadurch gekennzeichnet, dass** sie als alleinstehende Schraube, Pedikelschraube, Schenkelhalsschraube, als translaminarer Pin oder als Schraube zum Befestigen einer Platte vorgesehen ist.
